# EUROPEAN PATENT APPLICATION

(11) **EP 4 684 800 A1**
(43) Date of publication of application: **28.01.2026**
(21) Application number: 24775022.7
(22) Date of filing: 09.02.2024
(51) Int. Cl.: A61K 45/00, A61K 31/713, A61K 31/7088, A61K 48/00, A61P 9/10, A61P 25/00, C12N 15/113, G01N 33/15, G01N 33/50

(54) **PHARMACEUTICAL COMPOSITION FOR TREATING BRAIN INJURIES**

(30) Priority: 20.03.2023 JP 2023044551
(71) Applicant: INSTITUTE OF SCIENCE TOKYO, Tokyo 152-8550 (JP)
(72) Inventor: SHICHITA Takashi, Tokyo 152-8550 (JP); TSUYAMA Jun, Tokyo 152-8550 (JP)
(74) Representative: J A Kemp LLP
(86) International application number: PCT/JP2024/080014
(87) International publication number: WO 2024/195890

(57) **Abstract**

Provided is, for example, a drug capable of being used in the treatment of brain injury caused by cerebrovascular disease or traumatic brain injury. The present invention relates to, for example, a pharmaceutical composition comprising a compound that inhibits or suppresses the expression of *Zfp384* gene, or a compound that has inhibitory or suppressive activity against ZFP384 protein, or the like.

## Description

### Technical Field

The present invention relates to, for example, a pharmaceutical composition for the treatment of brain injury caused by cerebrovascular disease or traumatic brain injury.

### Background Art

Stroke, a type of brain injury caused by cerebrovascular diseases, is a major cause of serious sequela or a bedridden state all over the world, and the number of stroke patients is expected to increase as society ages. Under these circumstances, methods for treating stroke have been scarcely established. It is widely known that improvement in neurological symptoms or prognosis for life functions is expected by active rehabilitation within approximately several months after stroke onset. On the other hand, after 6 months from onset, even by active rehabilitation delivers no marked improvement in neurological symptoms, and the symptoms are likely to be established as sequela. Thus, a repair function in the brain declines with time after stroke onset (e.g., cerebral infarction) (for example, Non-Patent Literature 1).

With advances in single-cell RNA-seq analysis (transcriptome analysis) technology, diverse properties of glial cells constituting the brain, such as microglia, have received worldwide attention in recent years. The microglia are immune cells that reside in the central nervous system. These cells play an important role in inflammation after brain damage and, after a lapse of approximately 1 week after damage, gradually begin to exert neural repair functions that produce neurotrophic factors such as insulin-like growth factor (IGF1: insulin-like growth factor 1) (for example, Non-Patent Literature 2). However, it is totally unknown how reparative microglia are induced in brain tissues (initiation of repair) and what fate these cells acquire (loss of repair capacity). If this molecular mechanism can be elucidated, a revolutionary brain function recovery agent can be developed, which enhances reparative functions of microglia and permits sustention (prolongation) or resumption of a repair duration.

### Citation List

[Non-Patent Literature 1] Levard, D. et al. Filling the gaps on stroke research: Focus on inflammation and immunity. Brain Behav Immun 91, 649-667 (2021)
[Non-Patent Literature 2] Crotti, A. & Ransohoff, R.M. Microglial Physiology and Pathophysiology: Insights from Genome-wide Transcriptional Profiling. Immunity 44, 505-515 (2016)

### Summary of Invention

Under these circumstances, there has been a demand for the development of a novel drug or the like capable of being used in the treatment of brain injury caused by cerebrovascular disease, or traumatic brain injury.

The present invention has been made in view of such circumstances and provides the following pharmaceutical composition and the like.

(1) A pharmaceutical composition comprising a compound that inhibits or suppresses the expression of Zfp384 gene, or has inhibitory or suppressive activity against ZFP384 protein, or a prodrug thereof, or a pharmacologically acceptable salt of the compound or the prodrug, or a hydrate or a solvate of the compound, the prodrug, or the pharmacologically acceptable salt.
(2) The pharmaceutical composition according to (1), wherein the pharmaceutical composition is used in treatment of brain injury caused by cerebrovascular disease, or traumatic brain injury.
(3) The pharmaceutical composition according to (2), wherein the cerebrovascular disease is stroke, preferably cerebral infarction, brain hemorrhage, or subarachnoid hemorrhage.
(4) The pharmaceutical composition according to (2), wherein the treatment is recovery from neurological symptoms in brain injury, or sustention and/or resumption of the recovering effect.
(5) The pharmaceutical composition according to (1), wherein the compound that inhibits or suppresses the expression of *Zfp384* gene comprises a nucleic acid comprising a sequence complementary to at least a portion of a nucleotide sequence of the gene.
(6) The pharmaceutical composition according to (5), wherein the at least a portion of a nucleotide sequence of the gene comprises at least a portion of a nucleotide sequence encoding a DNA-binding domain of the ZFP384 protein.
(7) The pharmaceutical composition according to (1), wherein the compound that has inhibitory or suppressive activity against ZFP384 protein is one member or a combination of two or more members selected from a decoy nucleic acid, a compound that degrades the protein, and a compound that induces the degradation of the protein.
(8) A therapeutic agent for brain injury caused by cerebrovascular disease, or traumatic brain injury, comprising a compound that inhibits or suppresses the expression of *Zfp384* gene, or has inhibitory or suppressive activity against ZFP384 protein, or a prodrug thereof, or a pharmacologically acceptable salt of the compound or the prodrug, or a hydrate or a solvate of the compound, the prodrug, or the pharmacologically acceptable salt.
(9) A method for treating brain injury caused by cerebrovascular disease, or traumatic brain injury, the method comprising administering the pharmaceutical composition according to (1) or the therapeutic agent according to (8) to a subject having or suspected of having brain injury caused by cerebrovascular disease, or traumatic brain injury.
(10) A method for screening for a compound that inhibits or suppresses the expression of *Zfp384* gene, or a compound that has inhibitory or suppressive activity against ZFP384 protein, wherein the method comprises:
   adding a candidate compound to microglial cells capable of overexpressing the ZFP384 protein, and
   evaluating the candidate compound for whether the compound inhibits or suppresses the expression of *Zfp384* gene, or whether the compound has inhibitory or suppressive activity against ZFP384 protein.
(11) A pharmaceutical composition for treatment of brain injury caused by cerebrovascular disease, or traumatic brain injury, or a therapeutic agent for the brain injury, comprising a compound obtained by the screening method according to (10), or a prodrug thereof, or a pharmacologically acceptable salt of the compound or the prodrug, or a hydrate or a solvate of the compound, the prodrug, or the pharmacologically acceptable salt.

### Advantageous Effects of Invention

The present invention can provide, for example, a pharmaceutical composition capable of being used in treatment of brain injury caused by cerebrovascular disease, or traumatic brain injury, a therapeutic agent for the brain injury, a method for treating the brain injury, and a method for screening for a compound serving as an active ingredient for the pharmaceutical composition or the therapeutic agent.

### Brief Description of Drawings

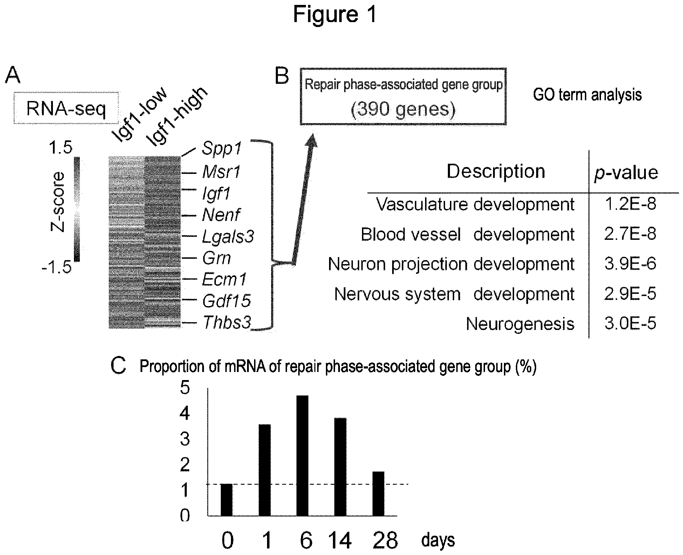

[Figure 1] Figure 1 is a diagram regarding the expression behavior of recovery phase-associated gene group in microglia after cerebral infarction.
   (A) A unique gene group of a recovery phase after cerebral infarction was defined as the recovery phase-associated gene group.
   (B) The recovery phase-associated gene group exhibited gene expression typical to neural repair such as vasculature and nervous tissue development.
   (C) The expression of the recovery phase-associated gene group after cerebral infarction in microglia was lost within 1 month.

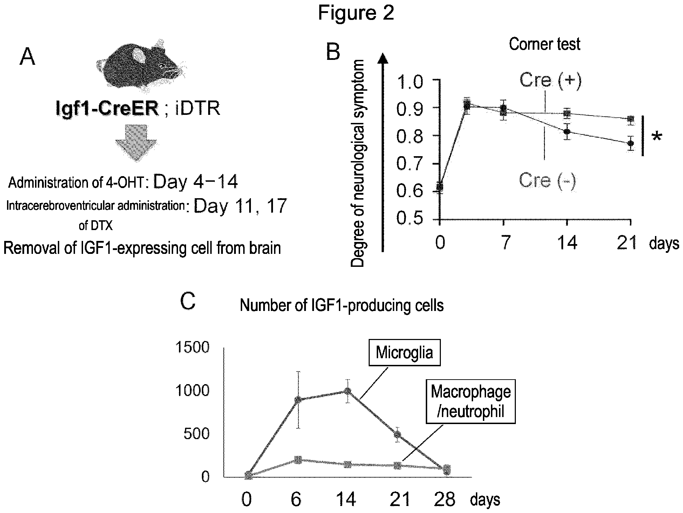

[Figure 2] Figure 2 is a diagram showing that microglia lose reparative functions by 28 days after cerebral infarction, and cells expressing IGF1 are responsible for functional recovery after cerebral infarction.
   (A) Cerebral infarction was generated using IGF1-CreER; iDTR mice, and IGF1-expressing cells were depleted from the brain by the administration of 4-OHT on 4 to 14 days after onset and the intracerebroventricular administration of DTX on 11 and 17 days after onset.
   (B) No recovery from neurological symptoms was observed in the mice from which the IGF1-expressing cells were depleted (Cre(+)) as compared with mice without depletion (Cre(-)).
   (C) The IGF1-expressing cells were mainly microglia and were no longer observed by 28 days after onset.
[Figure 3] Figure 3 is a diagram showing, by the fate tracing of reparative microglia, that microglia has lost reparative functions after cerebral infarction remain.
   When the fate of *Igf1*-expressing cells was traced, IGF1-EGFP-negative and TdTomato-positive cells were observed in the peri-infarct area 28 days after onset of cerebral infarction.
[Figure 4] Figure 4 is a diagram regarding the analysis of enhancer and promoter in microglia after cerebral infarction.
   (A) Chromatin loops that link open chromatin regions (promoter region and enhancer region) detected by ATAC-seq to a promoter and an enhancer detected by Hi-CHIP upstream and downstream of *Igf1* gene locus.
   (B) Time-dependent change after cerebral infarction in open chromatin regions detected by ATAC-seq upstream and downstream of recovery-associated genes.
   (C) Transcription factor candidates binding to open chromatin regions that changed from 14 to 28 days after cerebral infarction.

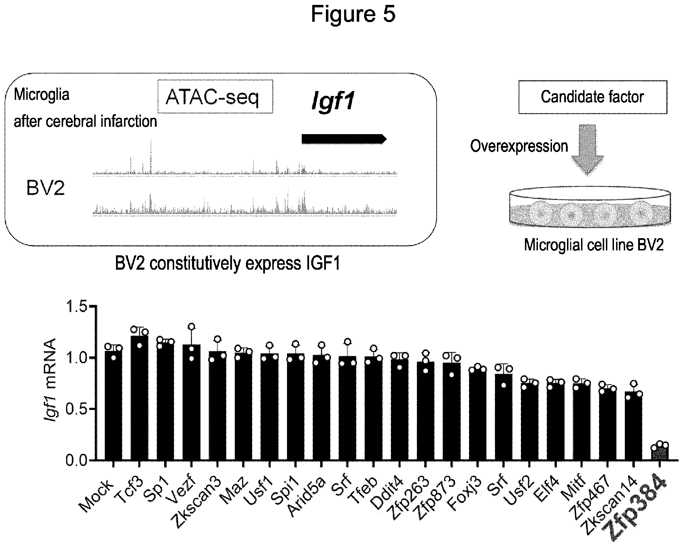

[Figure 5] Figure 5 is a diagram regarding the screening of a repair terminating factor. Among 21 potentially functional transcription factors in post-cerebral infarction microglia between day 14 and 28, *Zfp384* diminished the expression of *Igf1.*

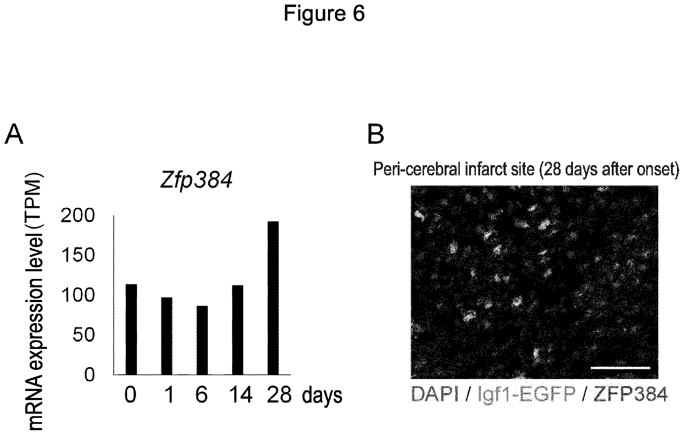

[Figure 6] Figure 6 is a diagram showing that the expression of *Zfp384* in microglia increases from 6 to 28 days after cerebral infarction.
   (A) Time-dependent change in microglial *Zfp384* expression after cerebral infarction.
   (B) Immunohistochemical staining showing that microglia expressing ZFP384 do not express IGF1.
[Figure 7-1]

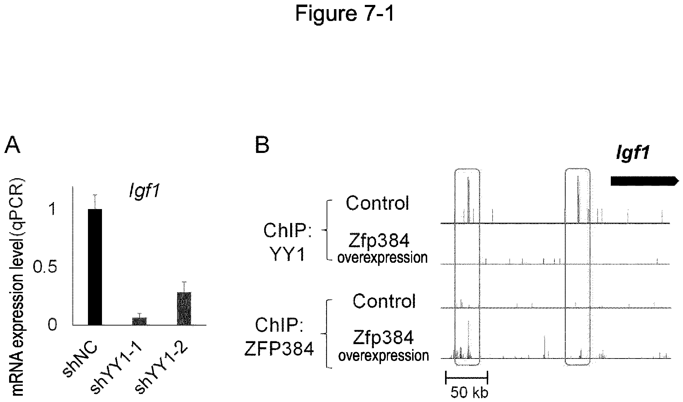

   Figure 7(A) is a diagram showing that decreased expression of YY1 in a microglial cell line, BV2, remarkably decreases the expression of *Igf1.*
   Figure 7(B) is a diagram showing that in the BV2 cell line, YY1 binds to an enhancer region upstream of the *Igf1* gene, whereas the overexpression of *Zfp384* removes the binding of YY1 so that ZFP384 instead binds.

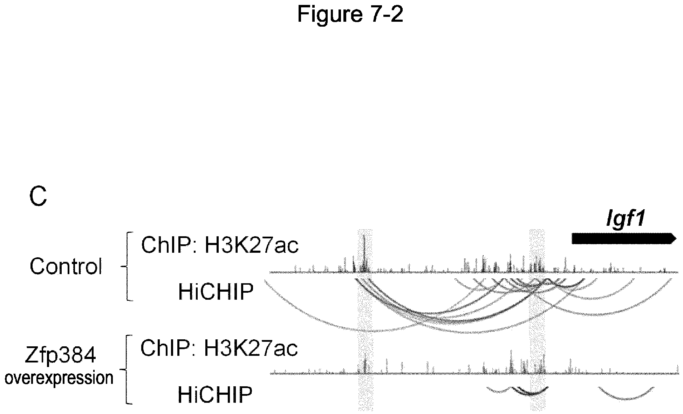

   Figure 7(C) is a diagram showing that the overexpression of *Zfp384* eliminates chromatin loops detectable by Hi-CHIP (H3K27ac is an open chromatin region).
   [Figure 7-2] (As described in Figure 7-1)
[Figure 8-1]

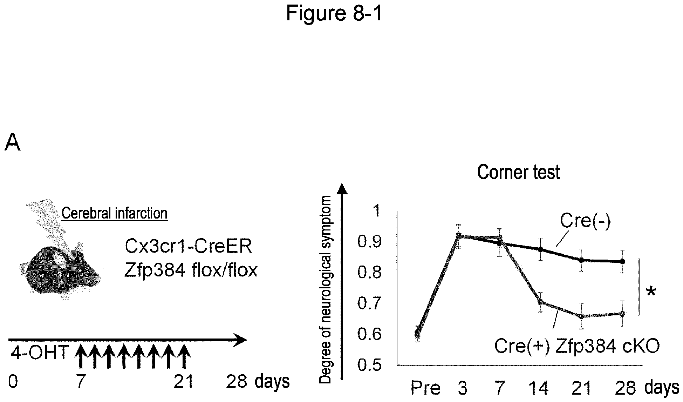

   Figure 8(A) is a diagram regarding the evaluation of long-term neurological symptoms after cerebral infarction by a corner test. Microglia-specific *Zfp384* deficiency on 7 days and later after cerebral infarction onset improved long-term neurological symptoms.

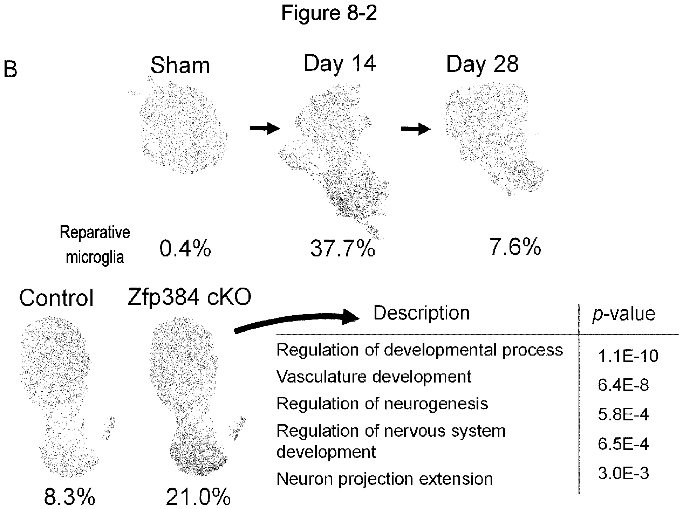

   Figure 8(B) is a diagram showing that microglia-specific *Zfp384* deficiency increased the proportion of reparative microglia expressing *Igf1* and increased the expression of gene groups involved in vasculature and nervous tissue development.
   [Figure 8-2] (As described in Figure 8-1)

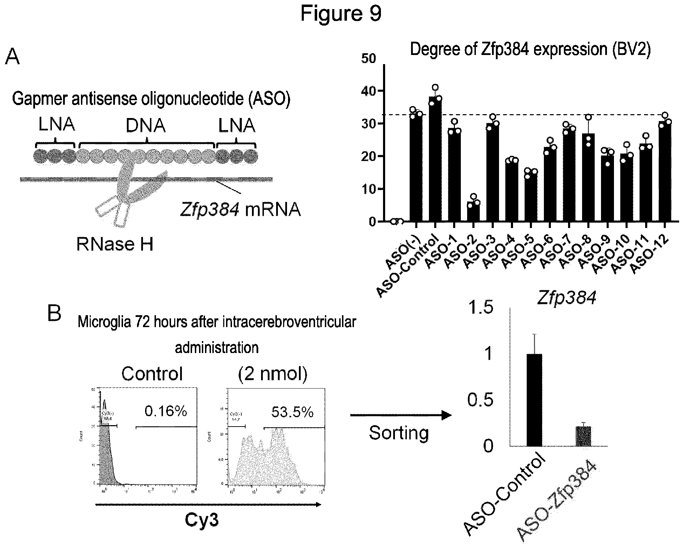

[Figure 9] Figure 9 is a diagram regarding, for example, the design of ASO targeting the *Zfp384* gene.
   (A) A schematic view of a gapmer antisense oligonucleotide (ASO), and the search for an ASO sequence that suppresses the expression of *Zfp384* with high efficiency in BV2 that overexpresses *Zfp384.*
   (B) Remarkable suppression of *Zfp384* expression was observed, mainly in microglia, by the intracerebroventricular administration of ASO-Zfp384.

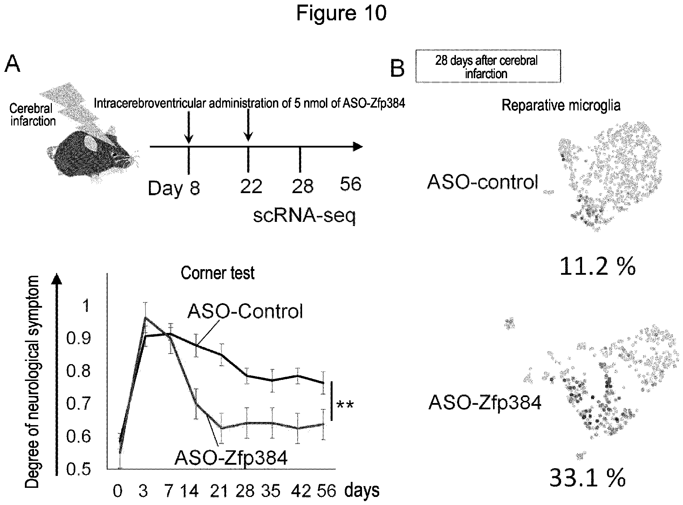

[Figure 10] Figure 10 is a diagram showing that the recovery from neurological symptoms was promoted and maintained by the administration of ASO-Zfp384. ASO-Zfp384 was found to sustain brain functional recovery.
   (A) ASO-Zfp384, even when administered on 8 days after cerebral infarction onset, significantly improved long-term neurological symptoms.
   (B) Microglia were isolated on 28 days after onset and analyzed by single-cell RNA-seq. As a result, the cell proportion of microglia expressing a recovery-associated gene was remarkably increased.

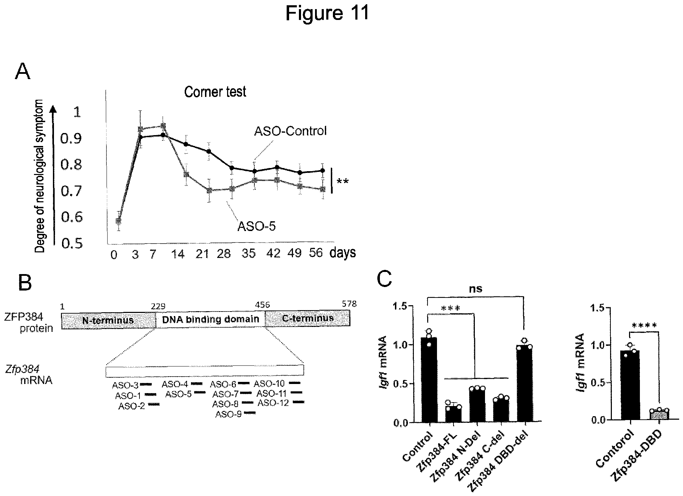

[Figure 11] Figure 11 is a diagram showing that ASO targeting a DNA binding domain of ZFP384 protein improves neurological symptoms after cerebral infarction.
   (A) ASO-5 administered on 8 days after cerebral infarction onset significantly improved long-term neurological symptoms.
   (B) The position of the sequence of each ASO designed against a DNA binding domain of ZFP384 protein and a mRNA sequence corresponding to the DNA binding domain.
   (C) The comparison of the degree of *Igf1* mRNA expression among BV2 cells made to overexpress mutant proteins of ZFP384. Zfp384-FL: overexpressing full-length ZFP384 protein, Zfp384 N-Del: ZFP384 protein lacking the N-terminal side, Zfp384 C-Del: ZFP384 protein lacking the C-terminal side, Zfp384 DBD-Del: ZFP384 protein lacking the DNA binding domain, and Zfp384-DBD: overexpressing only the DNA binding domain of the ZFP384 protein.

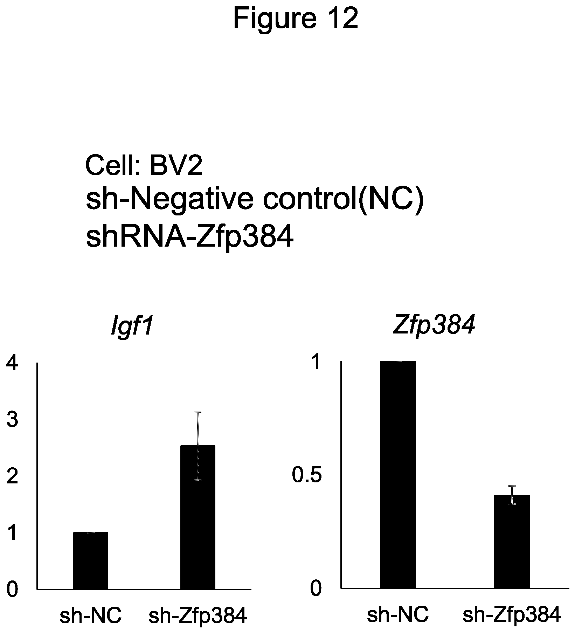

[Figure 12] Figure 12 is a diagram showing the change in the expression of *Igf1* mRNA and *Zfp384* mRNA in BV2 that expresses shRNA against *Zfp384.*

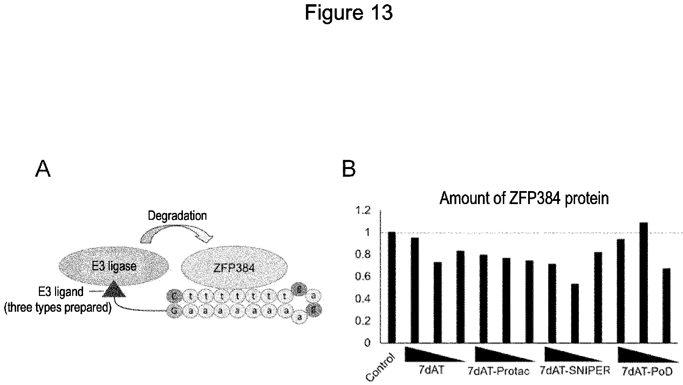

[Figure 13] Figure 13 is a diagram regarding the development of a Zfp384 inhibitor by a protein knockdown method.
   (A) A scheme showing the concept of the protein knockdown of ZFP384.
   (B) Protein knockdown drug screening in BV2. The concentrations are 100 nM, 300 nM, and 1 µM in order from left to right for each group.

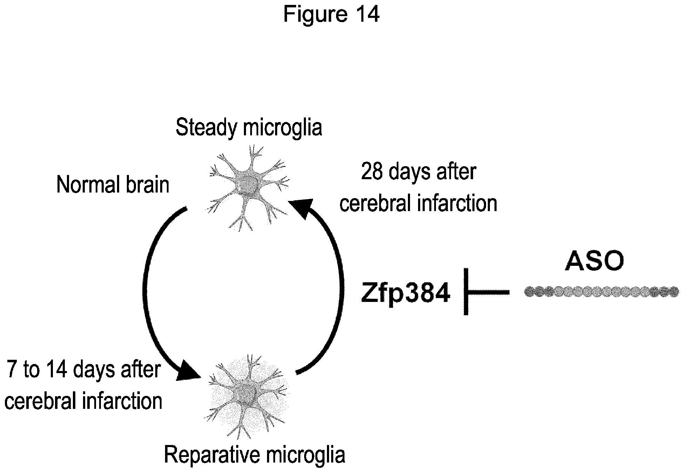

[Figure 14] Figure 14 is a schematic view summarizing Examples of the present application. Microglia continued to reside in the brain even after they lost reparative functions, and ZFP384 was discovered as a factor that caused loss of reparative functions in microglia after cerebral infarction. An antisense oligonucleotide that suppresses the expression of *Zfp384* gene successfully sustained and promoted functional recovery after cerebral infarction.

### Description of Embodiments

Hereinafter, the present invention will be described in detail. The scope of the present invention is not limited by the description, and embodiments other than those illustrated below can be carried out through appropriate changes or modifications made without departing from the spirit of the present invention. The present specification encompasses the whole specification of Japanese Patent Application No. 2023-044551 (filed on March 20, 2023), based on which the priority of the present application is claimed. All publications, for example, prior art documents, published bulletins, patent publications, and other patent literatures, cited herein are incorporated herein by reference.

### 1. Summary of the present invention

Cerebrovascular disease (stroke) is a major cause of the shortening of healthy life span, and currently, there are few approaches to recovering brain functions, other than rehabilitation. Since even rehabilitation is expected to attain remarkable functional recovery after stroke only within several months after onset, it is considered that reparative functions of brain cells are lost with time after stroke. Microglia are known to be responsible for a repairing effect when brain tissues are injured. The reparative functions or the cell fate of the microglia involved in the repair have not yet been elucidated. The present inventors have revealed that microglia, even after losing the repairing effect, remain in brain tissues, and successfully identified Zfp384 as a factor that diminishes reparative functions (repair terminating factor). Based on this achievement, the development of a compound (e.g., an antisense oligonucleotide) that suppresses the expression of the repair terminating factor has enabled, for the first time in the world, sustained recovery from neurological symptoms by maintaining a neural repair mechanism after cerebral infarction. The present invention provides a treatment strategy to sustain and/or resume functional recovery from neurological symptoms in patients with brain injury by sustaining (prolonging) the original repair mechanism possessed by the brain and thereby preventing the disabilities and sever neurological deficits.

### 2. Pharmaceutical composition, etc.

The pharmaceutical composition according to the present invention is characterized by comprising a compound that inhibits or suppresses the expression of *Zfp384* gene (gene encoding ZFP384 protein (zinc finger protein 384)), or a compound that has inhibitory or suppressive activity against ZFP384 protein (zinc finger protein 384), or a prodrug of the compound, or a pharmacologically acceptable salt of the compound or the prodrug, or a hydrate or a solvate of the compound, the prodrug, or the pharmacologically acceptable salt (hereinafter, sometimes simply referred to as a "Zfp384 inhibitor") as an active ingredient. As used herein, the simple term "Zfp384" also means the Zfp384 gene (*ZNF384* in human), and the simple term "ZFP384" also means the ZFP384 protein. The pharmaceutical composition according to the present invention is preferably, for example, but not limited to, a pharmaceutical composition that is used in the treatment of brain injury caused by cerebrovascular disease or traumatic brain injury.

In the present invention, examples of the brain injury caused by cerebrovascular disease as the brain injury to be treated include, but are not limited to, brain injury caused by stroke, and specifically include brain injury caused by cerebral infarction, brain hemorrhage, or subarachnoid hemorrhage.

In the present invention, examples of the treatment of brain injury caused by cerebrovascular disease, or traumatic brain injury specifically include recovery from neurological symptoms in brain injury, and sustention and/or resumption of the recovering effect, and additionally include suppression of progression and prognostic improvement of the brain injury.

The present invention can also encompass, for example, each aspect of the invention related to:
(i) a pharmaceutical composition for use in a method for treating brain injury caused by cerebrovascular disease, or traumatic brain injury in a test subject, the pharmaceutical composition comprising a Zfp384 inhibitor as an active ingredient;
(ii) a therapeutic agent for brain injury caused by cerebrovascular disease, or traumatic brain injury, comprising a Zfp384 inhibitor;
(iii) a method for treating brain injury caused by cerebrovascular disease, or traumatic brain injury, comprising using a Zfp384 inhibitor, specifically, for example, administering an effective amount of a Zfp384 inhibitor to a test subject;
(iv) use of a Zfp384 inhibitor for producing a drug for the treatment of brain injury;
(v) use of a Zfp384 inhibitor for treatment of the brain injury; and
(vi) a Zfp384 inhibitor for the treatment of brain injury.

The Zfp384 inhibitor serving as an active ingredient for the pharmaceutical composition, etc. of the present invention can be used after being uniquely synthesized, extracted, and purified, for example. The Zfp384 inhibitor is not limited, and a known or commercially available product may be used.

In the present invention, examples of the compound that inhibits or suppresses the expression of *Zfp384* gene as the Zfp384 inhibitor include, but are not limited to, a compound comprising a nucleic acid comprising a sequence complementary to at least a portion of a nucleotide sequence of the *Zfp384* gene, and preferably include a compound in which the at least a portion of a nucleotide sequence of the gene comprises at least a portion of a nucleotide sequence encoding a DNA binding domain of the ZFP384 protein. In this context, the mouse *Zfp384* gene has been registered and published, for example, under accession No: NM_001347452 on the website of GenBank of NCBI

### (National Center for Biotechnology Information)

(https://www.ncbi.nlm.nih.gov/genbank/). In the nucleotide sequence of the *Zfp384* gene (SEQ ID NO: 1), the nucleotide sequence encoding the DNA binding domain of the ZFP384 protein (SEQ ID NO: 2) is a sequence region from base positions 756 to 1265 in the sequence represented by SEQ ID NO: 1.

In the present invention, the nucleic acid comprising a sequence complementary to at least a portion of the nucleotide sequence (SEQ ID NO: 1) of the *Zfp384* gene is not limited by its type and may have a single-stranded structure or may have a double-stranded structure. Examples thereof include antisense nucleic acids (RNA degrading antisense nucleic acid (gapmer antisense nucleic acid), splicing controlling antisense nucleic acid, etc.), small interfering RNA (siRNA), and short hairpin RNA (shRNA). The base length of such a nucleic acid can be appropriately designed depending on the type and usage form of the nucleic acid and may be, for example, but not limited to, 5 to 50 bases, 6 to 24 bases, or 8 to 22 bases. The nucleic acid used in the present invention may be DNA or RNA, and nucleotides constituting the nucleic acid may be natural nucleotides, may be modified or artificial nucleotides (e.g., nucleotide mimics such as LNA (locked nucleic acid), peptide nucleic acid (PNA), phosphorothioate nucleic acid, and morpholino nucleic acid (morpholino oligo)), or may be a combination thereof.

The nucleic acid used in the present invention is not limited to the nucleic acid comprising a sequence complementary to at least a portion of the nucleotide sequence (SEQ ID NO: 1) of the Zfp384 gene. For example, a nucleic acid comprising a sequence having at least 60%, 70%, 80%, 85%, 90%, 95%, 96%, 97%, 98%, or 99% sequence identity to the complementary sequence is also preferably used.

The nucleic acid used in the present invention can be, for example, a nucleic acid modified (modified form) so as to improve the thermal stability of a complex with at least a partial sequence of the nucleotide sequence (SEQ ID NO: 1) of the target *Zfp384* gene.

Examples of the modified form include nucleic acids modified at the whole or a portion of bases (at least one base, or at least 10%, 20%, 30%, 40%, 50%, 60%, 70%, 80%, 90%, or 100% of the bases). Examples of the modified base include: 5-methylcytosine, 5-fluorocytosine, 5-bromocytosine, and 5-iodocytosine; 5-fluorouracil, 5-bromouracil, 5-iodouracil, and 5-hydroxyuracil; 2-thiothymine; N6-methyladenine and 8-bromoadenine; and N2-methylguanine and 8-bromoguanine.

Moreover, examples of the modified form include nucleic acids modified at sugar moieties of bases, i.e., sugar moieties of the whole or a portion of bases (at least one base, or at least 10%, 20%, 30%, 40%, 50%, 60%, 70%, 80%, 90%, or 100% of the bases). Examples thereof include nucleic acids having a structure where an arbitrary substance is added to the chemical structure of a sugar of a nucleotide or the chemical structure is substituted with an arbitrary substance. Such a modified sugar contained in the nucleic acid increases nuclease resistance. Examples of the modified sugar include bicyclic sugar, 5'-vinyl, 5'-methyl, 4'-S, 2'-F, 2'-OCH₃ (2'-methoxy or 2'-O-methyl group), and 2'-O(CH₂)₂OCH₃ substituents. The nucleic acid comprising the bicyclic sugar moiety is called bridged nucleotide (BNA). Examples of such a nucleic acid include methyleneoxy (4'-CH₂-O-2')-bridged sugar (LNA(R), 2',4'-BNA), ethyleneoxy (4'-(CH₂)₂-O-2')-bridged sugar (ENA), 4'-CH(CH₃)-O-2'-bridged sugar (cEt, constrained ethyl), 4'-CH(CH₂OCH₃)-O-2'-bridged sugar (cMOE, constrained MOE), and amide-bridged sugar (AmNA, amido-bridged nucleic acid).

Furthermore, the modified form may have a modification of the whole or a portion of phosphodiester bonds (at least one bond, or at least 10%, 20%, 30%, 40%, 50%, 60%, 70%, 80%, 90%, or 100% of the bonds) in the nucleic acid. Examples of the modification of the bonds include phosphorothioate bonds, phosphorodithioate bonds, phosphorodiamidate bonds, and phosphoramidate bonds. These modified bonds have higher nuclease resistance than that of naturally occurring bonds.

Examples of the nucleic acid used in the present invention specifically include antisense nucleic acids (ASO-1 to ASO-12) given below. Among them, ASO-2, ASO-4 to ASO-6, and ASO-9 to ASO-11 are preferred, ASO-2, ASO-4, and ASO-5 are more preferred, and ASO-2 is particularly preferred, from the viewpoint of excellent suppressive activity against the expression of the *Zfp384* gene. All the antisense nucleic acids (ASO-1 to ASO-12) given below are nucleic acids having 3-base LNA sequences added to both ends of a DNA sequence of 10 consecutive bases. The DNA is indicated in lower case "a, t, g, or c", and the LNA is indicated in upper case "A, T, or G" or "(L)" plus "5" (which means "5-methylcytosine"). The introduction position of the LNA in the antisense nucleic acid that may be used in the present invention is not limited to both ends of the DNA sequence as described above. If necessary, a portion or the whole of the LNA may be introduced, but not limited to, in the form of one base or a linkage of two or more bases between arbitrary bases in the DNA sequence. All the phosphodiester bonds between bases are phosphorothioated (indicated in "^"). A nucleic acid having bonds without the phosphorothioation between the whole or a portion of bases may be used as the nucleic acid for use in the present invention.

Moreover, the DNA sequence (10 bases) moiety in any of the antisense nucleic acids (ASO-1 to ASO-12) given below, or a nucleic acid sequence comprising the moiety may be used as the nucleic acid for use in the present invention. In this case, all the bonds between bases may be phosphorothioated as mentioned above, or bonds without the phosphorothioation may reside between a portion or the whole of bases, though the present invention is not limited thereto.

ASO-1: G(L)^G(L)^A(L)^t^t^t^c^t^c^a^c^a^g^A(L)^A(L)^G(L) (SEQ ID NO: 3)
ASO-2: G(L)^A(L)^A(L)^g^g^a^t^t^t^c^t^c^a^5(L)^A(L)^G(L) (SEQ ID NO: 4)
ASO-3: 5(L)^T(L)^5(L)^a^c^a^g^a^a^g^t^t^a^5(L)^A(L)^A(L) (SEQ ID NO: 5)
ASO-4: A(L)^5(L)^A(L)^t^t^t^g^t^a^t^g^g^t^5(L)^T(L)^A(L) (SEQ ID NO: 6)
ASO-5: G(L)^5(L)^A(L)^c^a^t^t^t^g^t^a^t^g^G(L)^T(L)^5(L) (SEQ ID NO: 7)
ASO-6: T(L)^G(L)^G(L)^a^g^a^g^t^t^g^t^g^t^G(L)^A(L)^A(L) (SEQ ID NO: 8)
ASO-7: G(L)^A(L)^T(L)^t^g^g^a^g^a^g^t^t^g^T(L)^G(L)^T(L) (SEQ ID NO: 9)
ASO-8: A(L)^G(L)^A(L)^t^t^g^g^a^g^a^g^t^t^G(L)^T(L)^G(L) (SEQ ID NO: 10)
ASO-9: 5(L)^A(L)^G(L)^a^t^t^g^g^a^g^a^g^t^T(L)^G(L)^T(L) (SEQ ID NO: 11)
ASO-10: G(L)^5(L)^A(L)^c^t^t^g^a^a^g^g^g^t^T(L)^T(L)^A(L) (SEQ ID NO: 12)
ASO-11: T(L)^G(L)^G(L)^c^a^c^t^t^g^a^a^g^g^G(L)^T(L)^T(L) (SEQ ID NO: 13)
ASO-12: G(L)^T(L)^G(L)^g^c^a^c^t^t^g^a^a^g^G(L)^G(L)^T(L) (SEQ ID NO: 14)

Bases in the sequence represented by SEQ ID NO: 1 complementary to the DNA sequence (10 bases) in each individual antisense nucleic acid (ASO-1 to ASO-12) described above are
in the case of ASO-1, bases from positions 929 to 944 in the sequence represented by SEQ ID NO: 1,
in the case of ASO-2, bases from positions 932 to 947 in the sequence represented by SEQ ID NO: 1,
in the case of ASO-3, bases from positions 923 to 938 in the sequence represented by SEQ ID NO: 1,
in the case of ASO-4, bases from positions 995 to 1010 in the sequence represented by SEQ ID NO: 1,
in the case of ASO-5, bases from positions 997 to 1012 in the sequence represented by SEQ ID NO: 1,
in the case of ASO-6, bases from positions 1035 to 1050 in the sequence represented by SEQ ID NO: 1,
in the case of ASO-7, bases from positions 1038 to 1053 in the sequence represented by SEQ ID NO: 1,
in the case of ASO-8, bases from positions 1039 to 1054 in the sequence represented by SEQ ID NO: 1,
in the case of ASO-9, bases from positions 1040 to 1055 in the sequence represented by SEQ ID NO: 1,
in the case of ASO-10, bases from positions 1085 to 1100 in the sequence represented by SEQ ID NO: 1,
in the case of ASO-11, bases from positions 1087 to 1102 in the sequence represented by SEQ ID NO: 1, and
in the case of ASO-12, bases from positions 1088 to 1103 in the sequence represented by SEQ ID NO: 1.

Examples of the nucleic acid used in the present invention also preferably include shRNA having the following sequence.

5'-
GCTCAGACTTTCCCTATTTTCAAGAGAAATAGGGAAAGTCTGAGCTGC-3'
(SEQ ID NO: 15)

In the shRNA, bases complementary to a sequence (3'-terminal 21 bases; AATAGGGAAAGTCTGAGCTGC) that works as siRNA in cells are bases from positions 471 to 491 (5'-GCAGCTCAGACTTTCCCTATT-3') in the sequence represented by SEQ ID NO: 1.

In the sequence of the shRNA, for example, but not limited to, at least a portion of the bases may be constituted by LNA.

The nucleic acid used in the present invention can be synthesized by, but not limited to, a known chemical synthesis method or enzymatic transcription method. Examples of the chemical synthesis method include phosphorothioate methods, phosphoramidite methods, and phosphotriester methods. Examples of the enzymatic transcription method include methods using RNA polymerase.

In the present invention, the compound that has inhibitory or suppressive activity against ZFP384 protein as the Zfp384 inhibitor can be designed or selected as, but not limited to, a compound capable of being used in a so-called protein knockdown technique. Examples thereof include decoy nucleic acids, compounds that degrade the protein, and compounds that induce the degradation of the protein, which may be appropriately used in combination. In this context, the presence or absence of the inhibitory or suppressive activity of the compound against the ZFP384 protein can be determined or confirmed, for example, by using a microglial cell line or, if necessary, a cell line made to overexpress exogenous *Zfp384* gene, and quantifying the amount of the ZFP384 protein by Western blotting or through a ChIP-seq (chromatin immunoprecipitation) method using an antibody against the ZFP384 protein.

The decoy nucleic acid is not limited, and a sequence (or a portion thereof) binding to the ZFP384 protein is preferably used. Examples thereof include a double-stranded sequence consisting of TTTTTTT-AAAAAAA (indicated by complementary strands connected with a hyphen "-"), and a sequence comprising the sequence.

Examples of the compound that degrades the ZFP384 protein include, but are not particularly limited to, various known or appropriately synthesized compounds that have the degrading activity.

Examples of the compound that induces the degradation of the ZFP384 protein include, but are not limited to, PROTAC, SNIPER, and cereblon E3 modulators.

For the Zfp384 inhibitor that may be used in the present invention, a derivative of the inhibitor may be used together with or instead of the inhibitor. The derivative can be a possible derivative of the inhibitor based on the technical common sense of those skilled in the art, and is not limited. The derivative also encompasses a derivative that undergoes metabolism, such as oxidation, reduction, hydrolysis, or conjugation in vivo, and also includes a compound (so-called prodrug) that undergoes metabolism, such as oxidation, reduction, or hydrolysis in vivo to produce the inhibitor or the derivative thereof. The derivative preferably has at least an equivalent degree of inhibition or suppression of the expression of *Zfp384* gene or an equivalent degree of inhibitory or suppressive activity against ZFP384 protein to that of the Zfp384 inhibitor.

A pharmacologically acceptable salt of such a compound or a prodrug may be used as the Zfp384 inhibitor that may be used in the present invention. Examples of the pharmacologically acceptable salt described above preferably include, but are not limited to, hydrohalides (e.g., hydrochloride, hydrobromide, and hydroiodide), inorganic acid salts (e.g., sulfate, nitrate, perchlorate, phosphate, carbonate, and bicarbonate), organic carboxylates (e.g., acetate, trifluoroacetate, maleate, tartrate, fumarate, and citrate), organic sulfonates (e.g., methanesulfonate, trifluoromethanesulfonate, ethanesulfonate, benzenesulfonate, toluenesulfonate, and camphor sulfonate), amino acid salts (e.g., aspartate and glutamate), quaternary amine salts, alkali metal salts (e.g., sodium salt and potassium salt), and alkaline earth metal salts (e.g., magnesium salt and calcium salt). Furthermore, the Zfp384 inhibitor may exist in the form of a hydrate or a solvate, and such a hydrate or a solvate may also be used as an active ingredient for the pharmaceutical composition and the therapeutic agent of the present invention.

In the pharmaceutical composition and the therapeutic agent of the present invention, the content ratio of the Zfp384 inhibitor as an active ingredient is not limited and can be appropriately set within a range that enables the inhibitor to inhibit or suppress the expression of the *Zfp384* gene or within a range that enables the inhibitor to have inhibitory or suppressive activity against the ZFP384 protein and further, in consideration of conditions such as a dosage form mentioned later. The content ratio can be, for example, within the range of 0.01 to 99% by weight with respect to the whole pharmaceutical composition or therapeutic agent, and may be within the range of 0.01 to 30% by weight, 0.05 to 20% by weight, or 0.1 to 10% by weight.

The administration route of the pharmaceutical composition and the therapeutic agent according to the present invention is not limited, and the pharmaceutical composition and the therapeutic agent can be administered through a route such as injection (intravenous injection, subcutaneous injection, intracutaneous injection, intramuscular injection, or injection into the joint cavity), transdermal administration (external use), transnasal administration, transmucosal administration, intranasal administration, intratracheal administration, oral administration, or inhalation as long as the Zfp384 inhibitor serving as an active ingredient can function in vivo (particularly, in brain tissues).

Examples of the dosage form of the pharmaceutical composition and the therapeutic agent according to the present invention include, but are not limited to, injections (including drips), inhalants, liquid formulations, emulsions, suspensions, solutions, ointments, nasal drops, tablets, granules, sprays, capsules, syrups, suppositories, tapes, and liposomes. The pharmaceutical composition and the therapeutic agent according to the present invention may contain a pharmacologically acceptable carrier, diluent, excipient, binder, disintegrant, lubricant, flavor, colorant, sweetener, corrigent, suspending agent, wetting agent, emulsifier, dispersant, aid, antiseptic, buffer, stabilizer, coating agent, or the like usually used in the pharmaceutical field. Examples of the carrier and the excipient include lactose, starch, talc, magnesium stearate, crystalline cellulose, methylcellulose, carboxymethylcellulose, glycerin, sodium alginate, and gum arabic. Examples of the binder include polyvinyl alcohol, polyvinyl ether, ethylcellulose, gum arabic, shellac, and saccharose. For example, physiological saline, or an isotonic liquid containing glucose or other aid drugs is used as an injectable aqueous liquid, which may be used in combination with an appropriate solubilizer, for example, an alcohol, a polyalcohol (e.g., propylene glycol), or a nonionic surfactant. For example, sesame oil or soybean oil is used as an oily liquid, which may be used in combination with a solubilizer such as benzyl benzoate or benzyl alcohol.

The dose of the pharmaceutical composition and the therapeutic agent according to the present invention can be a dose that attains a therapeutically effective amount of the Zfp384 inhibitor serving as an active ingredient. The therapeutically effective amount can be appropriately changed depending on the severity of a pathological condition, sex, age, body weight, or the like of a patient. For example, the Zfp384 inhibitor can be administered at a single dose of approximately 1 µg to approximately 2000 mg, more preferably approximately 10 µg to approximately 1000 mg, further preferably approximately 100 µg to approximately 500 mg, per kg of body weight of a patient. The administration can be performed once or several times (e.g., in four to six divided portions) a day, and intermittent administration may be performed at intervals of several days or several weeks.

The pharmaceutical composition and the therapeutic agent according to the present invention can be used in the treatment of brain injury caused by cerebrovascular disease or traumatic brain injury in any animal. The pharmaceutical composition and the therapeutic agent can be suitably used, particularly, for a mammal such as a human, a monkey, a bovine, a sheep, a horse, a dog, or a cat.

In the present invention, a kit comprising the Zfp384 inhibitor (as a specific example, a kit comprising the pharmaceutical composition or the therapeutic agent according to the present invention mentioned above) may be used for the treatment of brain injury caused by cerebrovascular disease or traumatic brain injury.

The form of the Zfp384 inhibitor in the kit is not limited, and the inhibitor may be contained in, for example, a dissolved state, in consideration of stability (preservation) and ease of use, etc.

The kit can appropriately contain other components, in addition to the Zfp384 inhibitor.

The kit should have at least the Zfp384 inhibitor mentioned above as a component. Thus, the kit may have, but is not limited to, all components essential for the treatment of brain injury caused by cerebrovascular disease, or traumatic brain injury, together with or separately from the Zfp384 inhibitor.

### 3. Screening method, etc.

The present invention provides, for example, a method for screening for a compound useful in the treatment of brain injury caused by cerebrovascular disease or traumatic brain injury.

Specifically, the present invention provides a method for screening for a compound that inhibits or suppresses the expression of *Zfp384* gene, or a compound that has inhibitory or suppressive activity against ZFP384 protein, characterized by comprising: adding a candidate compound to microglial cells capable of overexpressing the ZFP384 protein; and evaluating the candidate compound for whether the compound inhibits or suppresses the expression of *Zfp384* gene or whether the compound has inhibitory or suppressive activity against ZFP384 protein. The microglial cells capable of overexpressing the ZFP384 protein are not limited, and transformed cells can be used which are obtained by introducing the *Zfp384* gene to a known established microglial cell line (BV2 cells, etc.) by use of a known gene recombination technique. A candidate compound is added to the microglial cells in vitro, and cells expressing the ZFP384 protein (positive cells) can be quantified by, for example, FACS to evaluate the degree of inhibition or suppression of *Zfp384* gene expression by the candidate compound. Alternatively, after addition of the candidate compound, the expression level of the ZFP384 protein can be quantified by, for example, Western blot to evaluate the degree of inhibitory or suppressive activity against the ZFP384 protein (i.e., the degree of protein knockdown).

A compound obtained by the screening method described above can be used as an active ingredient for a pharmaceutical composition for the treatment of brain injury caused by cerebrovascular disease or traumatic brain injury, or a therapeutic agent for the brain injury. A prodrug of the obtained compound, or a pharmacologically acceptable salt of the compound or the prodrug, or a hydrate or a solvate of the compound, the prodrug, or the pharmacologically acceptable salt can also be used as the active ingredient in the same manner as above.

Hereinafter, the present invention will be described more specifically with reference to Examples. However, the present invention is not limited by these Examples.

### Examples

In Examples of the present application, the contents, etc. summarized below will be described.

Example 1: It was found that the formation of chromatin conformation was important for the expression of a recovery-associated gene group in microglia after cerebral infarction. The expression and enhancer interaction of the recovery-associated gene group disappeared in the murine model of cerebral infarction on 28 days after onset (which corresponds to 90 days after cerebral infarction onset in humans), whereas the microglia involved in the repair were confirmed to remain in brain tissues, though their reparative functions were dormant.

Example 2: ZFP384 was identified as a transcription factor diminishing the repair capacity (recovery-associated gene expression) of microglia. It was demonstrated that the suppression of *Zfp384* expression in microglia after cerebral infarction can prolong the expression of recovery-associated genes and improve long-term neurological symptoms after cerebral infarction.

Example 3: Antisense oligonucleotides (nucleic acid therapeutics) that suppress the expression of *Zfp384* were constructed. It was demonstrated that these antisense oligonucleotides, even when their administration was started after a lapse of 7 days or thereafter from cerebral infarction onset, are capable of sustaining the expression of recovery-associated genes in microglia and improving long-term neurological symptoms.

### [Material and method, etc.]

Prior to the description of each Example, materials and methods, etc. used in the present Examples will be described below.

### <Cerebral ischemic model>

8- to 24-week-old mice were used. Embolization filaments with their tips coated with silicone (ETHILON (thread size: 7-0), Johnson & Johnson) were inserted through the common carotid artery of each of the 8- to 24-week-old mice. The brain temperature was maintained at 36°C. Cerebral blood flow was monitored with Doppler blood flow meter ALF21 (ADVANCE CO., LTD.), and the embolization filaments were indwelled to the origin of the middle cerebral artery such that cerebral blood flow in the middle cerebral artery perfusion area was maintained at 20 to 40% of the steady state. Reperfusion of brain tissues was facilitated by withdrawing the embolization filaments 60 minutes after ischemia. Sham-operated mice were prepared as a control (Figures 1 to 4, 6, 8, 10, and 11).

### <Neurological symptom after induction of cerebral ischemia>

Neurological symptoms were evaluated over time using evaluation scores by the corner test reported by Schallert T et al. (Neuropharmacology. 2000 Mar 3; 39 (5): 777-87) (Figures 2, 8, 10, and 11).

### <Immunostaining>

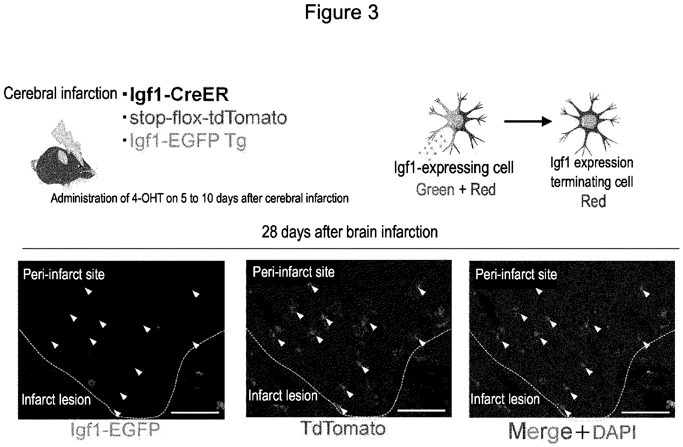

The murine model of cerebral ischemia was transcardially perfused with 4% paraformaldehyde to fix the brain tissues 28 days after onset. The brain was excised and then sliced into 10 to 15 µm sections, followed by immunohistochemical staining. The primary antibodies used were an anti-ZFP384 antibody (Sigma-Aldrich Co., LLC, Cat.# HPA004051) (1:300 dilution) and an anti-EGFP antibody (Aves Labs, Inc., Cat.# 1010) (1:500 dilution). The secondary antibodies used were an Alexa 488-labeled secondary antibody (anti-chicken IgG, Thermo Fisher Scientific Inc., Cat.# A-11039) (1:300 dilution) and an Alexa 546-labeled antibody (anti-rabbit IgG, Thermo Fisher Scientific Inc., Cat.# A-11081) (1:300 dilution). The secondary antibodies and dihydrochloride (DAPI) (DOJINDO LABORATORIES, Cat.# SE196) (1:200 dilution) were used for nuclear staining. The scale bars of immunostaining images represent 100 µm (Figures 3 and 6).

### <Preparation of lentivirus>

HEK293 cells were transfected with a lentivirus vector (CSII-EF, RIKEN) with each transcription factor cloned thereinto, together with a packaging plasmid pMDL g/p-RRE and an envelope plasmid pCMV-VSV-G-RSV-Rev, using polyethyleneimine, and 72 hours later, a supernatant was centrifuged overnight at 8000 g at 4°C to collect lentivirus (Figure 5).

### <Functional analysis of transcription factor>

A microglial cell line BV2 was infected with the lentivirus, and 48 hours later, gene expression analysis was conducted by qPCR described above (Figure 5).

### <shRNA>

shRNA having the following sequence was synthesized and used.

GCTCAGACTTTCCCTATTTTCAAGAGAAATAGGGAAAGTCTGAGCTG
C (SEQ ID NO: 15)

The sequence was cloned into a lentivirus vector (CS-EG-H1, RIKEN). Lentivirus was prepared and collected by the same method as in the lentivirus for transcription factor expression (Figure 12).

### <Protein knockdown>

A double-stranded sequence TTTTTTT-AAAAAAA serving as a sequence binding to the DNA binding domain of the ZFP384 protein was bound with E3 ligase VHL (von Hippel Lindau) through an amide-PEG3-amine bond, and the resultant was used as PROTAC. An IAP (inhibitor of apoptosis protein) antagonist A 410099.1 was bound with a nucleic acid through amide-PEG3-amine, and the resultant was used as SNIPER. Pomalidomide was bound with a nucleic acid using 4'-PEG2-Amine. The synthesis of these three compounds for protein knockdown was requested to Ajinomoto Bio-Pharma (Figure 13).

### <Antisense oligonucleotide (ASO) and Zfp384 knockout>

The synthesis of a designed sequence of ASO was requested to Ajinomoto Bio-Pharma, and the resultant was purchased and used. For the functional evaluation of ASO, the ASO was added at a concentration of 1 µM to BV2 that overexpressed Zfp384-P2A-mCherry using lentivirus, and mCherry-positive cells were evaluated by FACS quantification (Figure 9). For the knockout of *Zfp384, Zfp384* was knocked out in microglia by the intraperitoneal administration of 4-hydroxytamoxifen (Sigma-Aldrich Co., LLC, Cat.# H6278) at 10 mg/kg to *Cx3cr1-CreER; Zfp384*^{flox/flox} mice at frequencies of once two days from 7 to 21 days after induction of cerebral ischemia (Figure 8). The administration of the ASO to the mice was carried out by the intracerebroventricular administration of the ASO diluted with PBS at 5 nmol/10 µL to the mice (Figures 10 and 11).

### <Isolation of microglia>

A live cell fraction was collected by Percoll density gradient from brain tissues perfused with saline, and fluorescently immunostained with an anti-CD45 antibody (eBioscience, Cat.# 11-0451-85) (30-F11) (1:400 dilution) and an anti-CD11b antibody (eBioscience, Cat.# 45-0112-82) (M1/70) (1:400 dilution), and microglia were isolated by use of FACS (Figures 2, 4, and 6). When *Igf1-Egfp* transgenic mice were used, the anti-CD45 antibody used was APC-labeled 30-F11 (BioLegend, Inc., Cat# 103112) (1:400 dilution) (Figure 3).

### <Gene expression in microglia and BV2>

RNA was extracted using RNeasy Micro Kit (Qiagen N.V.) or RNAiso (Takara Bio Inc.), and the gene expression of interest was quantitatively evaluated by quantitative PCR after reaction mediated by reverse transcriptase. In addition, RNA-seq libraries were prepared using Ovation SoLo RNA-Seq Library Preparation Kit (Tecan Trading AG) (Figures 1 and 5 to 12). For the analysis of RNA-seq data, STAR (Dobin A et al., Bioinformatics. 2013 Jan 1; 29 (1): 15-21) was used in mapping to the mouse mm10 genome, and reads mapped onto mRNA were normalized by the transcripts per million (TPM) method and used as gene expression levels. For comprehensive functional analysis of gene expression, extracted characteristic genes were functionally analyzed using a public database g:profiler (https://biit.cs.ut.ee/gprofiler/) (Figures 1, 8, and 10).

### <ATAC-seq library>

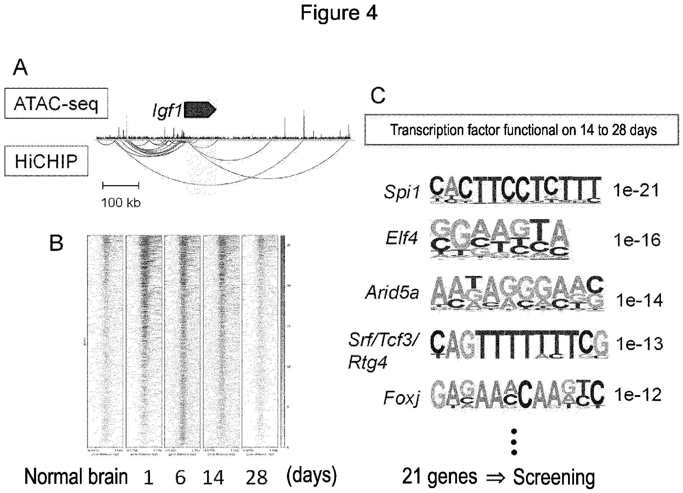

ATAC-seq libraries were prepared from microglia isolated by FACS on the basis of the approach of Buenrostro JD et al. (Nat Methods., 2013 Dec; 10 (12): 1213-8) using Tagment DNA TDE1 Enzyme and Buffer Kits (Illumina, Inc.) (Figures 4 and 5). ATAC-seq data were mapped to mouse mm10 genome using bowtie2 (Langmead B et al., Nat Methods., 2012 Mar 4; 9 (4): 357-9) and normalized by the reads per genome coverage (RPGC) method. The peak call of ATAC-seq was carried out by Genrich (https://github.com/jsh58/Genrich). For HiCHIP, cells were fixed in 1% formaldehyde. Then, the genome was cleaved with a restriction enzyme MboI (New England Biolabs Japan, Cat #. R0147), and cleaved ends were filled using biotinylated ATP to generate blunt ends. Then, ligation and ultrasonication were performed, and chromatin immunoprecipitation was performed using an anti-H3K27ac antibody (Abcam plc, Cat.# ab4729) (1:100 dilution). Libraries were constructed by the approach of Mumbach MR et al. (Nat Genet., 2017 Nov; 49 (11): 1602-1612) (Figures 4 and 7). For the analysis of Hi-CHIP data, default parameters of HiC-Pro pipeline (Servant N et al., 2015, Genome Biology volume 16, Article number: 259) were used in mapping to the mouse mm10 genome, and loops were detected by FitHiCHIP (Bhattacharyya S et al. Nat Commun., 2019 Sep 17; 10 (1): 4221). Enhancer regions that interacted with promoters of the repair phase-associated gene group were detected by Hi-CHIP. The chromatin accessibility of the enhancer regions was extracted by ATAC-seq, and a heat map was then prepared using deepTools (Ramirez F et al., Nucleic Acids Research., Volume 44, Issue W1, 8, 2016). For motif analysis, enriched transcription factor motifs were extracted using findMotifsGenome.pl of HOMER software (http://homer.ucsd.edu/homer/index.html) (Figure 4). For ChIP-seq, cells were permeabilized with 5% digitonin. Then, an anti-ZFP384 antibody (Sigma-Aldrich Co., LLC, Cat.# HPA004051) or an anti-YY1 antibody (Abcam plc, Cat.# ab109237) (EPR4652) was diluted 100-fold and added to the cells. After overnight shaking, libraries were prepared by the CUT&Tag method (Kaya-Okur HS et al., Nat Commun., 2019 Apr 29; 10 (1): 1930) (Figure 7). For single-cell RNA-seq (scRNA-seq), libraries were prepared using Chromium Next GEM Single Cell 5' Reagent Kits v2 purchased from 10x Genomics, Inc. scRNA-seq data were mapped using cellranger-6.0.1 and visualized by Loupe Browser (10x Genomics, Inc.) and Seurat (https://satijalab.org/seurat/) (Figures 8 and 10).

### [Example 1]

### <Expression of recovery-associated gene in microglia is induced via chromatin conformation, and microglia remain in brain even after losing reparative function>

(1) In order to analyze the gene group characteristics of microglia on 6 days and later after onset, which was a recovery phase of neurological symptoms after cerebral infarction, comprehensive gene expression analysis was conducted by RNA-seq. The expression of a recovery phase-associated gene group in microglia was induced after cerebral infarction, peaking 6 to 14 days after onset, and decreased to a level similar to that in the normal brain by 28 days after onset. Microglia highly expressing IGF1 in a cerebral infarct lesion were isolated using *Igf1*-EGFP reporter mice, and 390 genes that were highly expressed in these reparative microglia and exhibited higher expression than that in microglia of the normal brain were identified as a recovery phase-associated gene group. In order to examine the expression behavior of the recovery phase-associated gene group, post-cerebral infarction day 1, 6, 14, or 28 microglia were isolated, and gene expression profiles were analyzed by RNA-seq. The results revealed that the expression of the recovery phase-associated gene group exhibited a peak on 6 days after cerebral infarction and was no longer observed (reparative functions were lost) 28 days after cerebral infarction (Figure 1).
(2) In order to analyze the significance of cells expressing IGF1 in a recovery phase after cerebral infarction, *Igf1-CreERT2* knock-in mice were prepared and crossed with mice expressing diphtheria toxin receptor (iDTR) by the administration of 4-hydroxy tamoxifen (4-OHT). When diphtheria toxin (DTX) was intracerebroventricularly administered to the mice given 4-OHT, IGF1-producing cells in the brain were depleted. As a result, no recovery from neurological symptoms after cerebral infarction was observed (Figure 2: Cre(+)), revealing that IGF1-expressing cells play an important role in functional recovery after cerebral infarction. Although the IGF1-expressing cells after cerebral infarction were mainly microglia, the microglia expressing IGF1 were no longer observed by 28 days after onset (Figure 2). (Cre(+) n = 24, Cre(-) n = 18) (* p < 0.05, two-way ANOVA with Tukey's post-hoc test).
(3) The cell fate of microglia that have lost reparative functions has not been known so far. Accordingly, *Igf1-CreERT2* knock-in mice that permitted fate tracing of cells expressing *Igf1* were prepared and crossed with stop-flox-tdTomato and *Igf1-*EGFP mice to trace the cell fate of reparative microglia. As a result, microglia in a state that lost reparative functions, albeit expressing *Igf1* in the past, were confirmed to exist at the peri-infarct area 28 days after cerebral infarction onset (Figure 3). These cells have been confirmed to be Iba1-positive cells showing their microglial identity. These results demonstrated that reparative microglia still remain in the brain even after losing reparative functions.

### [Example 2]

### <Microglia-specific deficiency of Zfp384 identified as a repair-terminating factor provides sustained recovery from neurological symptoms in cerebral infarction mice over long period>

(1) From the fact that microglia that have lost reparative functions remain in brain tissues, the presence of a factor diminishing the repair capacity of microglia was predicted. Accordingly, in order to reveal an expression control mechanism of the recovery phase-associated gene group, ATAC-seq and Hi-ChIP were carried out by which genome-wide identification of enhancer-promoter interaction mediated by chromatin loops was achieved. As a result, the number of interacting enhancers was increased in the promoter region of the recovery-associated gene group after cerebral infarction onset so that a marked number of chromatin loops were formed. Nonetheless, almost the same chromatin status as that of the normal brain was found 28 days after cerebral infarction onset (Figure 4). These results were consistent with the course of the gene expression behavior obtained by RNA-seq. It was indicated that the enhancer interaction mediated by chromatin loops was important for the expression of the recovery phase-associated gene group in microglia after cerebral infarction.
(2) Next, transcription factors functional in microglia 14 to 28 days after cerebral infarction onset were comprehensively detected as transcription factor motifs binding to open chromatin regions (DNA sequences) obtained by ATAC-seq (Figure 4). As a result, 21 transcription factors were obtained as candidates, among which a repair-terminating factor was presumably included. Therefore, a repair terminating factor that suppressed the expression of *Igf1* was screened for by using a microglial cell line BV2 constitutively expressing IGF1, and allowing the cells to overexpress the candidate factors included in the transcription factor motifs. As a result, the overexpression of *Zfp384* was found to remarkably suppress the expression of *Igf1* (Figure 5) (n = 3).
(3) The expression of *Zfp384* in microglia was found to increase from 6 to 28 days after cerebral infarction onset (Figure 6). The expression of ZFP384 and IGF1 was observed by qPCR and immunostaining using *Igf1-*EGFP mice. As a result, microglia expressing ZFP384 was found to express no IGF1.
(4) For the formation of chromatin loops, proteins such as CTCF, YY1, and RAD21 are known to bind to genomic DNA and create promoter-enhancer interaction. These proteins were searched for a factor that influenced the expression of the *Igf1* gene. As a result, it was found that YY1 was important for the expression of the *Igf1* gene (Figure 7). The analysis of YY1- and ZFP384-bound regions by CHIP-seq using a microglial cell line BV2 was performed and revealed that YY1 bound to the enhancer region of the *Igf1* gene in BV2 constitutively expressing *Igf1,* whereas the binding of YY1 was canceled in BV2 that overexpressed *Zfp384* so that ZFP384 instead bound thereto. The overexpression of *Zfp384* eliminated chromatin loops observed around the *Igf1* gene locus. These results indicate that increased expression of ZFP384 decreases the expression of recovery-associated genes such as IGF1.
(5) Microglia-specific *Zfp384*-deficient mice (*Cx3cr1-CreERT2; Zfp384* flox mice) were created to examine cerebral infarction models. Microglial deficiency of *Zfp384* in the mice by the administration of 4-OHT 7 days or later after cerebral infarction was found to significantly improve neurological symptoms over a long period as compared with a control group without the deficiency of *Zfp384* (Figure 8) (Cre(+) n = 24, Cre(-) n = 18) (* p < 0.05, two-way ANOVA with Tukey's post-hoc test).

Microglia were isolated from brain tissues 28 days after cerebral infarction onset and analyzed by single-cell RNA-seq. As a result, reparative microglia highly expressing *Igf1* were more increased in the microglia-specific *Zfp384*-deficient mice than in wild type (control) (8.3% vs 21.0%). Factors involved in angiogenesis or nervous tissue construction were enriched in a gene group whose expression increased in reparative microglia (Figure 8). These results indicated that the suppression of *Zfp384* expression enables microglia having reparative functions to be maintained in the brain.

### [Example 3]

### <Antisense oligonucleotide, etc. targeting Zfp384 sustain brain functional recovery brought about by microglia>

(1) The results of each preceding Example suggested that a therapeutic drug that sustains recovery processes after cerebral infarction can be developed by the drugs that inhibit ZFP384. Accordingly, an antisense oligonucleotide (ASO) that suppresses or inhibits the expression of the Zfp384 gene was developed and studied for its therapeutic effect on murine models of cerebral infarction. A chimeric nucleic acid of phosphorothioated DNA and LNA (locked nucleic acid) as a nucleic acid structure was found to remarkably suppress the expression of *Zfp384.* 12 types of various ASOs (ASO-1 to ASO-12 (SEQ ID NOs: 3 to 14)) were designed so as to be complementary to mRNA of the *Zfp384* gene. For details of the sequence of each ASO, see the above description of the specification of the present application. As a result of adding each ASO (ASO-1 to ASO-12) to a microglial cell line BV2 that overexpressed Zfp384, all the ASOs were capable of suppressing, albeit to different degrees, the expression of the *Zfp384* gene. Among them, ASO-2, ASO-4 to ASO-6, and ASO-9 to ASO-11 had a high suppressive effect on *Zfp384* gene expression. Particularly, ASO-2 (hereinafter, referred to as "ASO-Zfp384") most effectively suppressed *Zfp384* gene expression (Figure 9). When this ASO-Zfp384 was fluorescently labeled with Cy3 and intracerebroventricularly administered, the ASO was found to be incorporated, mainly in microglia, with high efficiency, and found to remarkably suppress the expression of *Zfp384.*
(2) In the case of intracerebroventricularly administering ASO-Zfp384 to cerebral infarction mouse models, this ASO, even when administered on 8 days after onset, was confirmed to have a remarkable improving effect on neurological symptoms (Figure 10) (ASO-con n = 7, ASO-Zfp384 n = 7) (** p < 0.01, two-way ANOVA with Tukey's post-hoc test). Results of single-cell RNA-seq analysis on post-cerebral infarction day 28 microglia revealed that microglia expressing recovery-associated genes can be maintained in the brain. Specifically, ASO-Zfp384 attains recovery of long-term brain functions by blocking loss of reparative functions and sustaining neural repair. As seen from these results, a revolutionary brain function recovery agent (repair agent for long-term neurological symptoms in brain tissues) can be developed with a nucleic acid therapeutic targeting *Zfp384.*
(3) An approach of suppressing the expression of *Zfp384* was studied. For example, ASO-5 (Figure 9), which had the second strongest suppressive effect on the expression of *Zfp384* in BV2 to ASO-Zfp384 (ASO-2), was administered to a murine model of cerebral infarction. As a result, ASO-5 produced an improving effect on neurological symptoms over a long period to a degree next to ASO-Zfp384 (Figure 11) (ASO-con (control ASO; n = 7), ASO-5 (n = 4)) (** p < 0.01, two-way ANOVA with Tukey's post-hoc test). This suggested that the degree of improvement in neurological symptoms after cerebral infarction may vary depending on the strength of suppression of *Zfp384* expression. The examination of ASO that suppresses *Zfp384* expression in BV2, as shown in Figure 9, was carried out for the mRNA sequence corresponding to the DNA-binding domain of the ZFP384 protein (Figure 11). This is because the DNA-binding domain of the ZFP384 protein plays an important role in decreasing the expression of recovery-associated genes such as IGF1. Specifically, remarkably decreased expression of IGF1 was observed in BV2 cells that expressed the full-length ZFP384 protein or a mutant protein lacking the N-terminus or the C-terminus of ZFP384. By contrast, these cells, when made to express a mutant protein of ZFP384 lacking the DNA binding domain, had no influence on the expression of IGF1 (n = 3) (*** p < 0.001, one-way ANOVA with Dunnet's post-hoc test). Remarkably decreased expression of IGF1 was observed in BV2 that overexpressed only the DNA binding domain of the ZFP384 protein (n = 3) (**** p < 0.0001, two-sided t-test).
(4) On the other hand, a *Zfp384* expression suppressive effect and an Igf1 expression enhancing effect were also observed in BV2 made to express shRNA against Zfp384 (GCTCAGACTTTCCCTATTTTCAAGAGAAATAGGGAAAGTCTGAGCTGC (SEQ ID NO: 15)) (Figure 12) (n = 2). Therefore, such shRNA is included in approaches applicable to the treatment of brain injury, such as cerebral infarction. A suppressive effect on the expression of ZFP384 protein in BV2 was examined by the protein knockdown method using an E3 ligase ligand bound with a DNA motif sequence to which the ZFP384 protein could bind. The ZFP384 protein is a transcription factor that binds to double-stranded DNA having the sequence TTTTT-AAAAA. Accordingly, it was believed that the ZFP384 protein could be selectively degraded by adding a small molecule binding to E3 ubiquitin ligase capable of inducing ubiquitination and proteasomal degradation of a protein, to an oligonucleotide (7dAT) (5'-CTTTTTTTGAGAAAAAAAAG-3' (SEQ ID NO: 16)) capable of working as a decoy for the ZFP384 protein. Three types of E3 ligase ligands were prepared: VHL ligand known as PROTAC, cIAP ligand known as SNIPER, and a thalidomide derivative pomalidomide. 7dAT or E3 ligase ligand-bound 7dAT was added to a microglial cell line BV2 and collected 72 hours later, and the amount of the ZFP384 protein was quantified by Western blot. As a result, in the sample supplemented with E3 ligase ligand-bound 7dAT, a tendency to decrease the amount of the ZFP384 protein was observed, though no concentration-dependent effect was observed. The tendency to decrease the amount of the ZFP384 protein was actually observed, suggesting that the protein knockdown method is also an approach applicable to the treatment of brain injury such as cerebral infarction (Figure 13) (n = 1).

### [Discussion]

The present Examples revealed the cell fate of microglia that exert reparative functions, and a revolutionary nucleic acid that sustains and promotes reparative functions (drug candidate) was found from the discovery of the transcription factor ZFP384 that caused loss of reparative functions. In the present Examples, functional recovery after cerebral infarction was successfully maintained by suppressing the effect of ZFP384 and maintaining a chromatin status responsible for neural repair in microglia. An antisense oligonucleotide (ASO) as well as every nucleic acid therapeutic that suppresses the expression of *Zfp384* and inhibits its effect, such as an artificial nucleic acid that works as a decoy for ZFP384, is expected to be designed or developed as an approach of suppressing the effect of ZFP384. In addition to such nucleic acid therapeutics, a low-molecular compound that has a Zfp384 inhibitory effect, and a compound designed so as to specifically promote the degradation of the ZFP384 protein, for example, are expected to be developed. Examples of the advantages of the nucleic acid therapeutics include long-term pharmacological effects that can be expected even at a small number of doses, and incorporation into microglia with relatively high selectivity to exhibit pharmacological effects. A possible approach for human patients involves, for example, intraspinally administering a drug by lumbar puncture, and allowing the drug to reach the ventricle depending on the specific gravity of the drug. Similar approaches are generally used in cerebral ventriculography to be executed for normal pressure hydrocephalus patients. In addition, the administration of the nucleic acid therapeutics by lumbar puncture has already been carried out for spinal muscular atrophy patients. Such a treatment strategy can be applied to cerebrovascular disease (stroke) such as cerebral infarction and brain hemorrhage as well as central nervous trauma.

In previous reports, concepts or Examples are few in number in which a nucleic acid therapeutic is used and allowed to act on microglia to develop a therapeutic drug. ASO-Zfp384 developed by the present inventors were able to have a long-term effect on microglia in the brain by intracerebroventricular administration. The present inventors examined a number of ASOs and determined a target sequence for effective suppression of *Zfp384* expression. In the present Examples, intracerebroventricular administration of the most effective ASO to mice on 8 days after cerebral infarction onset was performed and confirmed to sustain and maintain a repairing effect and improve neurological symptoms on 28 to 56 days after onset (which correspond to 3 to 6 months after onset in cerebral infarction patients) (Figure 14). A treatment strategy to sustain brain functional recovery over a long period using ASO-Zfp384 or the like is very revolutionary compared to modern medicine, which is composed mainly of rehabilitation for brain injury patients.

### Industrial Applicability

The present invention can provide, for example, a pharmaceutical composition capable of being used in treatment of brain injury caused by cerebrovascular disease, or traumatic brain injury, a therapeutic agent for the brain injury, a method for treating the brain injury, and a method for screening for a compound serving as an active ingredient for the pharmaceutical composition or the therapeutic agent.

### Free Text of Sequence Listing

SEQ ID NOs: 3 to 16: synthetic oligonucleotides

## Claims

1. A pharmaceutical composition comprising a compound that inhibits or suppresses the expression of *Zfp384* gene, or has inhibitory or suppressive activity against ZFP384 protein, or a prodrug thereof, or a pharmacologically acceptable salt of the compound or the prodrug, or a hydrate or a solvate of the compound, the prodrug, or the pharmacologically acceptable salt.

2. The pharmaceutical composition according to claim 1, wherein the pharmaceutical composition is used in treatment of brain injury caused by cerebrovascular disease, or traumatic brain injury.

3. The pharmaceutical composition according to claim 2, wherein the cerebrovascular disease is stroke, preferably cerebral infarction, brain hemorrhage, or subarachnoid hemorrhage.

4. The pharmaceutical composition according to claim 2, wherein the treatment is recovery from neurological symptoms in brain injury, or sustention and/or resumption of the recovering effect.

5. The pharmaceutical composition according to claim 1, wherein the compound that inhibits or suppresses the expression of *Zfp384* gene comprises a nucleic acid comprising a sequence complementary to at least a portion of a nucleotide sequence of the gene.

6. The pharmaceutical composition according to claim 5, wherein the at least a portion of a nucleotide sequence of the gene comprises at least a portion of a nucleotide sequence encoding a DNA binding domain of the ZFP384 protein.

7. The pharmaceutical composition according to claim 1, wherein the compound that has inhibitory or suppressive activity against ZFP384 protein is one member or a combination of two or more members selected from a decoy nucleic acid, a compound that degrades the protein, and a compound that induces the degradation of the protein.

8. A therapeutic agent for brain injury caused by cerebrovascular disease, or traumatic brain injury, comprising a compound that inhibits or suppresses the expression of *Zfp384* gene, or has inhibitory or suppressive activity against ZFP384 protein, or a prodrug thereof, or a pharmacologically acceptable salt of the compound or the prodrug, or a hydrate or a solvate of the compound, the prodrug, or the pharmacologically acceptable salt.

9. A method for treating brain injury caused by cerebrovascular disease, or traumatic brain injury, the method comprising administering the pharmaceutical composition according to claim 1 or the therapeutic agent according to claim 8 to a subject having or suspected of having brain injury caused by cerebrovascular disease, or traumatic brain injury.

10. A method for screening for a compound that inhibits or suppresses the expression of *Zfp384* gene, or a compound that has inhibitory or suppressive activity against ZFP384 protein, wherein the method comprises:
adding a candidate compound to microglial cells capable of overexpressing the ZFP384 protein, and
evaluating the candidate compound for whether the compound inhibits or suppresses the expression of *Zfp384* gene, or whether the compound has inhibitory or suppressive activity against ZFP384 protein.

11. A pharmaceutical composition for treatment of brain injury caused by cerebrovascular disease, or traumatic brain injury, or a therapeutic agent for the brain injury, comprising a compound obtained by the screening method according to claim 10, or a prodrug thereof, or a pharmacologically acceptable salt of the compound or the prodrug, or a hydrate or a solvate of the compound, the prodrug, or the pharmacologically acceptable salt.
